# EUROPÄISCHE PATENTSCHRIFT

(11) **EP 2 815 695 B1**
(45) Veröffentlichungstag und Bekanntmachung des Hinweises auf die Patenterteilung: **19.06.2019**
(21) Anmeldenummer: 13173012.9
(22) Anmeldetag: 20.06.2013
(51) Int. Cl.: A61B 5/00, A61B 18/14

(54) **Chirurgisches Instrument mit Gewebeerkennung**
Surgical instrument with tissue detection
Instrument chirurgical doté d'une reconnaissance de tissus

(43) Veröffentlichungstag der Anmeldung: 24.12.2014
(60) Teilanmeldung: 19161757.0
(73) Patentinhaber: Erbe Elektromedizin GmbH, 72072 Tübingen (DE)
(72) Erfinder: Spether, Dominik, 79102 Freiburg (DE); Fischer, Klaus, 72202 Nagold (DE); Neugebauer, Alexander, 72116 Mössingen (DE)
(74) Vertreter: Rüger Abel

(56) Entgegenhaltungen:
- EP-A1- 2 514 380
- WO-A2-2011/055369
- US-A1- 2008 221 409

## Beschreibung

Die Erfindung betrifft eine elektrochirurgische Einrichtung zur Einwirkung auf ein biologisches Gewebe mittels elektrischer Entladung. Insbesondere betrifft die Erfindung eine elektrochirurgische Einrichtung mit HF-Skalpell für die offenchirurgische oder laparoskopische Chirurgie.

Aus der WO 2011/055369 ist die Plasmaablation mittels zu einer Elektrode geleiteter HF-Leistung zur Plaquebeseitigung in Blutgefäßen bekannt. Das entsprechende Instrument wird in das Blutgefäß eingeführt und erzeugt an seinem distalen Ende einen HF-Funken, der auf Plaques oder auch auf anderes biologisches Material wie Blut einwirkt. Das entstehende Licht wird einem optischen Spektrometer zugeleitet, das anhand des Vorhandenseins oder nicht Vorhandenseins der typischen Phosphor-Spektrallinie Information darüber liefert, ob lebende Zellen oder Plaque von dem Funken aufgelöst werden.

Eine elektrochirurgische Einrichtung nach der Präambel von Anspruch 1 ist aus WO2011055369 bekannt.

Prinzipiell ist es somit für den Anwender möglich, durch entsprechende maschinelle Unterstützung die chirurgisehe Aktion auf bestimmte biologische Strukturen, hier die Plaques, zu konzentrieren und anderes Gewebe zu schonen.

Es ist Aufgabe der Erfindung, dem Anwender bei der Benutzung HF-chirurgische Instrumente eine verbesserte Orientierung zu ermöglichen.

Diese Aufgabe wird mit der elektrochirurgischen Einrichtung nach Anspruch 1 gelöst:
Die erfindungsgemäße elektrochirurgische Einrichtung umfasst ein Instrument mit einer Elektrode, die mit einer elektrischen Quelle verbindbar ist. Das Instrument kann als offenchirurgisches monopolares oder bipolares Instrument ausgebildet sein. Ebenso kann es als laparoskopisches monopolares oder bipolares Instrument ausgebildet sein. Es weist somit zumindest eine Elektrode auf, die mit HF-Leistung speisbar ist. Eine den Stromkreis schließende zweite Elektrode kann eine am Patienten zu befestigenden Neutralelektrode oder eben eine zweite Elektrode am Instrument sein.

Zu der Einrichtung gehört eine Lichtaufnahmeeinrichtung, die vorzugsweise in Bezug auf die Elektrode ortsfest angeordnet, d.h. mit dieser an einem Instrument vorgesehen ist. Die zumindest eine (erste) Lichtaufnahmeeinrichtung legt ein erstes Lichtaufnahmefeld fest, das zumindest einen Teil des von der Elektrode erzeugten Funkens erfasst. Die Lichtaufnahmeeinrichtung ist zum Beispiel über eine Glasfaser oder ein Glasfaserbündel mit einer Lichtanalyseeinrichtung zur Ermittlung von Gewebemerkmalen durch Spektralanalyse verbunden. Die Lichtanalyseeinrichtung ist darauf eingerichtet, Merkmale des aufgenommenen Spektrums mit Referenzdaten zu vergleichen und daraus eine Aussage abzuleiten, ob die elektrische Entladung, d.h. zum Beispiel der HF-Funken, mit einem bestimmten Gewebetyp in Berührung steht oder nicht in Berührung steht. Diese Information wird an eine Indikatoreinrichtung gegeben. Die Indikatoreinrichtung kann als akustische Indikatoreinrichtung ausgebildet sein. Dazu kann sie einen ein- oder mehrtönigen Signalgeber aufweisen. Dieser kann an dem Instrument, z.B. an seinem Handgriff, oder auch an dem speisenden Gerät (HF-Generator), an der Lichtanalyseeinrichtung angeordnet oder als gesonderter Signalgeber ausgebildet sein. Ein solcher kann über Kabel oder eine kabellose Übertragungsstrecke mit der Lichtanalyseeinrichtung verbunden sein.

Vorzugsweise ist die Indikatoreinrichtung als optische Indikatoreinrichtung mit optischen Indikatoren ausgebildet. Vorzugsweise ist diese Indikatoreinrichtung so angeordnet, dass das vom Anwender wahrzunehmende optische Signal im Anwendungssichtfeld, d.h. in einem während der Anwendung vom Anwender sichtbaren Bereich liegt.

Bei einem offenchirurgischen Instrument kann die Indikatoreinrichtung durch eine oder mehrere Indikatoren in Gestalt von Leuchten am Handgriff des Instruments, beispielsweise an seinem distalen Ende gebildet sein. Alternativ kann sowohl bei einem offenchirurgischen Instrument wie auch bei einem laparoskopischen Instrument die Indikatoreinrichtung Lichtaustrittsfenster aufweisen, die Licht auf das von der Elektrode berührte Gewebe projizieren. Bei einem laparoskopischen Instrument kann die Indikatoreinrichtung außerdem oder alternativ darauf eingerichtet sein, entsprechende Informationen in ein von dem Anwender wahrgenommenes Bild, beispielsweise auf einer Videobrille oder einem Bildschirm einzublenden. Die Anzeige kann durch verschiedene Lichtfarben, durch verschiedene Symbole oder die Position von Farben oder Symbolen auf einer Projektionsfläche gegeben sein. Die Projektionsfläche kann das biologisehe Gewebe oder ein Videobildschirm sein. Beispielsweise kann die Indikatoreinrichtung zur Anzeige der Gewebemerkmale verschiedene Lichtfarben anzeigen, beispielsweise grünes Licht bei Normalgewebe, rotes Licht bei Tumorgewebe oder blaues Licht bei unbekanntem Gewebe. Das Licht kann durch Lichtleitfasern auf das Anwendungsfeld geleitet werden und so dem Anwender eine leichte Orientierung ermöglichen.

Die insoweit beschriebenen Grundprinzipien können mit einem Instrument verwirklicht werden, das lediglich eine Lichtaufnahmeeinrichtung und somit nur ein (erstes) Lichtaufnahmefeld aufweist. Vorzugsweise ist die Einrichtung jedoch mit einer zweiten Lichtaufnahmeeinrichtung versehen, die ein zweites Lichtaufnahmefeld festlegt, das sich von dem ersten Lichtaufnahmefeld unterscheidet. Damit kann an dem Instrument nicht nur Information über die Beschaffenheit des von der elektrischen Entladung betroffenen Gewebes gewonnen werden, sondern zusätzlich Information über die Richtung der Lage einer Gewebegrenze relativ zu der Elektrode. Beispielsweise können die Lichtaufnahmefelder so festgelegt sein, dass sie ihren Mittelpunkt von der Elektrode aus gesehen, in unterschiedlichen Richtungen aufweisen. Die Lichtaufnahmefelder können dabei einander überschneidend oder, wie es bevorzugt wird, einander nicht überschneidend ausgebildet sein. Es können damit in verschiedenen Zonen des HF-Funkens auftretende unterschiedliche Leuchterscheinungen, die auf die Berührung des räumlich ausgedehnten HF-Funkens mit zwei verschiedenen Gewebetypen zurückgehen, zugleich oder nacheinander erfasst und daraus eine Richtungsinformation abgeleitet werden. Diese kann dazu dienen, verschiedene im Sichtfeld des Anwenders liegende Indikatoren zu aktivieren oder zu deaktivieren. Die Aktivierung oder Deaktivierung kann im Aufleuchten oder Verlöschen der Indikatoren oder in einem Farbumschlag derselben bestehen oder durch Anzeige bzw. Nichtanzeige von Symbolen erfolgen.

Zur Ermittlung von Gewebemerkmalen durch Spektralanalyse vergleicht die Lichtanalyseeinrichtung das von dem Gewebe gelieferte Spektrum mit einem Referenzspektraldatensatz. Vorzugsweise wird der Referenzspektraldatensatz in einem oder mehreren Kalibrierschritten gewonnen. Ein Kalibrierschritt wird vorzugsweise mit reduziertem Strom an bekanntem Gewebe durchgeführt. Zum Beispiel kann zu Beginn der Anwendung ein HF-Funke mit einem interessierenden Gewebetyp, beispielsweise gesundem Gewebe und/oder auch krankhaft verändertem Gewebe, mit so geringem Strom erzeugt werden, dass zwar eine Leuchterscheinung entsteht, ein chirurgischer Effekt jedoch unterbleibt. Damit wird sichergestellt, dass die Kalibrierung, d.h. die Aufnahme mindestens eines Referenzspektraldatensatzes ohne Schädigung von Gewebe vorgenommen wird.

Das erfindungsgemäße Instrument kann Lichtaustrittsfenster und Lichteintrittsfenster aufweisen. Beispielsweise können die Lichtaustrittsfenster durch distale Enden (Stirnflächen) von zur Signalabgabe dienenden Lichtleitfasern gebildet sein, während die Lichteintrittsfenster durch die distalen Enden (Stirnflächen) von zur Lichtaufnahme dienenden Lichtleitfasern gebildet sind. Die lichtaufnehmenden und lichtabgebenden Fasern können in einem oder mehreren Strängen oder Bündeln angeordnet sein. Den Lichteintrittsfenstern (und/oder Lichtaustrittsfenstern) kann eine Reinigungseinrichtung zugeordnet sein. Diese kann durch Düsen zum Austritt von Reinigungsfluiden, d.h. Flüssigkeiten oder Gasen, gebildet sein. Der aus diesen Düsen z.B. parallel zur Schneidelektrode austretende Gas oder Flüssigkeitsstrom kann zur Temperierung der Lichtaus- und/oder -eintrittsfenster dienen, um einen Kondenswasserniederschlag ebenso zu verhindern wie das Ankleben von Gewebe. Vorzugsweise ist in räumlicher Nähe zu der oder den Gasaustrittsdüsen, z.B. im Handgriff des Instruments, ein Ventil zur gesteuerten Freigabe des Gas- oder Fluidstroms untergebracht. Das Ventil kann elektrisch oder mechanisch mit einem Aktivierungsschalter verbunden sein, der der elektrischen Aktivierung der Elektrode dient. Für die Freigabe des Fluids kann eine Vorlaufzeit und ein Nachlaufzeit vorgesehen sein, um sicher zu stellen, dass der Fluidstrom in gewünschter Stärke vorhanden ist, sobald und solange die Elektrode aktiviert ist.

Das Instrument kann an seinem distalen Ende einen abnehmbaren Teil aufweisen, der zum lediglich einmaligem Gebrauch vorgesehen ist, während das übrige Instrument sterilisierbar und zum mehrmaligen Gebrauch vorgesehen ist. Die Elektrode ist dazu an einem Kupplungsstück gehalten, das mit dem Instrument lösbar verbunden ist. Außerdem weist das Kupplungsstück mindestens ein Sichtfenster auf, das zum Beispiel ringförmig um die Elektrode herum angeordnet sein kann. Dies gilt sowohl für laparoskopische als auch für offenchirurgische Instrumente.

Bevorzugterweise weist die Lichtanalyseeinrichtung eine Betriebssteuereinrichtung auf, die dazu eingerichtet ist, die spektrale Gewebeanalyse nur dann zu starten, wenn die Lichtanalyseeinrichtung eine festgelegte Spektraleigenschaft erfasst. Die festgelegte Spektraleigenschaft kann beispielsweise darin bestehen, dass das erfasste Spektrum innerhalb eines vorgegebenen Toleranzbands liegt. Damit kann die Betriebssteuereinrichtung erkennen, ob das aufgenommene Licht überhaupt von biologischem Gewebe bzw. einer mit diesem in Berührung stehenden elektrischen Entladung herrührt. Ein nicht mit biologischem Gewebe in Berührung stehender Funke oder auch Umgebungslicht erkennt die Betriebssteuereinrichtung hingegen als irrelevant und löst somit keine Gewebeanzeige aus. Auf diese Weise kann die erfindungsgemäße Einrichtung als Nachrüsteinrichtung für bestehende elektrochirurgische Geräte angeboten werden. Eine elektrische Verbindung zwischen dem speisenden Gerät ist nicht erforderlich.

Weitere Einzelheiten vorteilhafter Ausführungsformen der Erfindung sind Gegenstand der Beschreibung, der Ansprüche oder der Zeichnung. Es zeigen:
Figur 1 die erfindungsgemäße Einrichtung, in schematischer Darstellung,
Figur 2 das Instrument aus Figur 1, in schematisierter ausschnittsweiser Perspektivdarstellung.
Figur 3 das Instrument nach Figur 2, in schematisierter Seitenansicht.
Figur 4 Lichterfassungsfelder des Instruments nach Figur 2, in schematisierter Darstellung.
Figur 5 eine Ausführungsform des erfindungsgemäßen Instruments, in schematisierter ausschnittsweiser Längsschnittdarstellung.
Figur 6 eine abgewandelte Ausführungsform des Instruments nach Figur 2, in einer schematisierten ausschnittsweisen Längsschnittdarstellung.
Figur 7 das Instrument nach Figur 5, in einer schematisierten Stirnansicht.
Figur 8 das Instrument nach Figur 6, in einer schematisierten Stirnansicht.
Figur 9 eine abgewandelte Ausführungsform eines erfindungsgemäßen Instruments, in schematisierter Stirnansicht.
Figur 10 eine Faseranordnung für sendende und empfangende Fasern für das Instrument nach Figur 8 oder 9.
Figur 11 einen Faserkoppler zur Zusammenführung bzw. Trennung von sendender und empfangender Lichtleitfaser für ein Instrument nach Figur 7.
Figur 12 ein Instrument mit besonders reduziertem zur einmaligen Verwendung vorgesehenen Kupplungsstück, in schematisierter längs geschnittener Darstellung.
Figur 13 eine besonderes einfache Version eines Instruments, in schematisierter längs geschnittener ausschnittsweiser Darstellung.
Figur 14 eine abgewandelte Ausführungsform eines erfindungsgemäßen Instruments mit Lichtaufnahmeeinrichtung, in schematisierter ausschnittsweiser Längsschnittdarstellung.
Figur 15 und 16 verschiedene Prismenanordnungen an der Lichtaufnahmeeinrichtung zur Festlegung des Lichtaufnahmefelds.
Figur 17 bis 20 verschiedene Ausführungsformen von Lichtaufnahmeeinrichtungen an erfindungsgemäßen Instrumenten zur Festlegung unterschiedlicher Lichtfelder.
Figur 21 Lichtaufnahmefelder an einem Instrument mit drei Lichtaufnahmeeinrichtungen.
Figur 22 das Instrument in drei verschiedenen Positionen bezüglich einer Gewebegrenze, unter schematischer Veranschaulichung der entsprechenden Anzeige bzw. Indikation.
Figur 23 die Grundstruktur der Lichtanalyseeinrichtung als Blockschaltbild.
Figur 24 die Grundstruktur einer abgewandelten Ausführungsform einer Lichtanalyseeinrichtung.
Figur 25 die Grundstruktur einer Verarbeitungseinrichtung zur Lichtanalyse mit Betriebssteuereinrichtung zum Start und Stopp der Gewebeanalyse und
Figur 26 Zeitdiagramme zur Veranschaulichung einer Kalibrierung mit reduziertem chirurgischen Effekt.

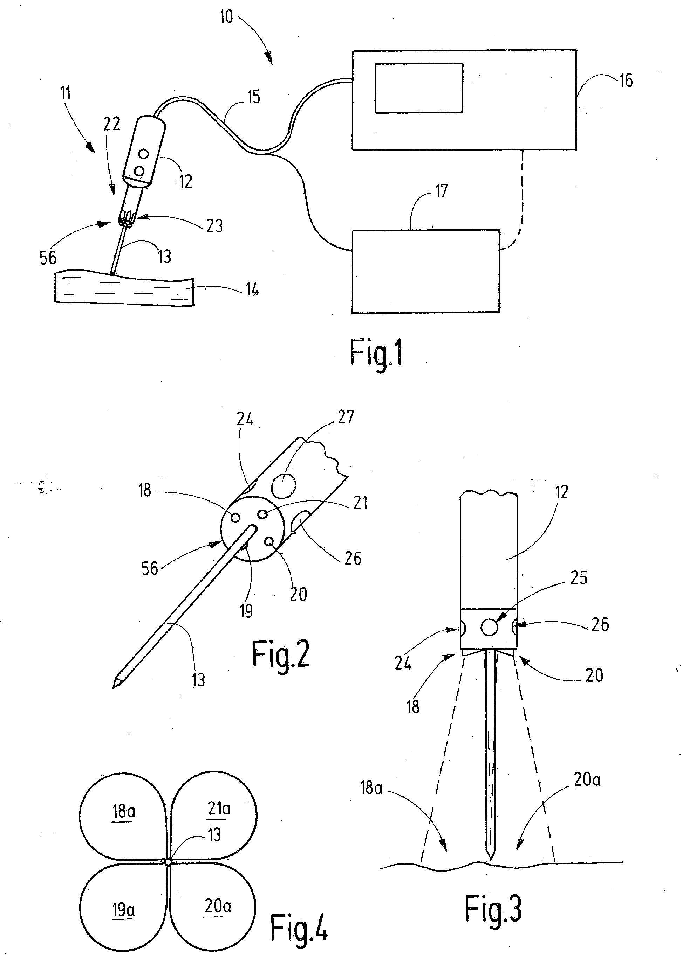

In Figur 1 ist eine Einrichtung 10 für die Elektrochirurgie schematisch veranschaulicht. Zu der Einrichtung 10 gehören ein Instrument 11 mit einem Handgriff 12 und einer Elektrode 13 zur Einwirkung auf Gewebe 14. Das Instrument 11 ist über ein Kabel 15 mit einem speisenden Gerät 16, zum Beispiel in Gestalt eines HF-Generators verbunden. Außerdem führt das Kabel 15 oder ein Teil desselben zu einer Lichtanalyseeinrichtung 17, mittels dessen an der Elektrode 13 durch elektrische Entladung entstehendes Licht analysiert wird, um den berührten Gewebetyp zu bestimmen. Die Lichtanalyseeinrichtung 17 führt dazu eine Spektralanalyse des im HF-Funken der Elektrode 13 erzeugten Lichts durch. Dieses wird über eine Lichtaufnahmeeinrichtung 56 aufgenommen, die mindestens ein, vorzugsweise aber mehrere Lichteintrittsfenster 18, 19, 20, 21 umfasst, die in der Nähe der Elektrode 13, beispielsweise um diese herum angeordnet sind. Das über die Lichteintrittsfenster 18 bis 21 aufgenommene Licht wird der Lichtanalyseeinrichtung 17 beispielsweise durch in dem Kabel 15 angeordnete Lichtleitfasern zugeleitet und spektral analysiert. Es dient zur Erkennung des Gewebetyps und zur Erzeugung entsprechender Signale, die vorzugsweise über eine im Anwendungssichtfeld 22 angeordnete Indikatoreinrichtung 23 abgegeben werden. Das Anwendungssichtfeld ist dabei derjenige Bereich in der Nähe des Handgriffs 12 und der Elektrode 13, der bei der Anwendung vom Anwender einsehbar ist und bei der Anwendung im Blickfeld des Anwenders liegt.

Die Indikatoreinrichtung 23 schafft die Möglichkeit, beispielsweise während eines elektrochirurgischen Eingriffs direkt am Patienten eine durchgeführte Gewebeanalyse schnell und mit hoher Aussagekraft anzuzeigen. Dadurch ist die Möglichkeit der Differenzierung von Geweben während einer laufenden Anwendung gegeben.

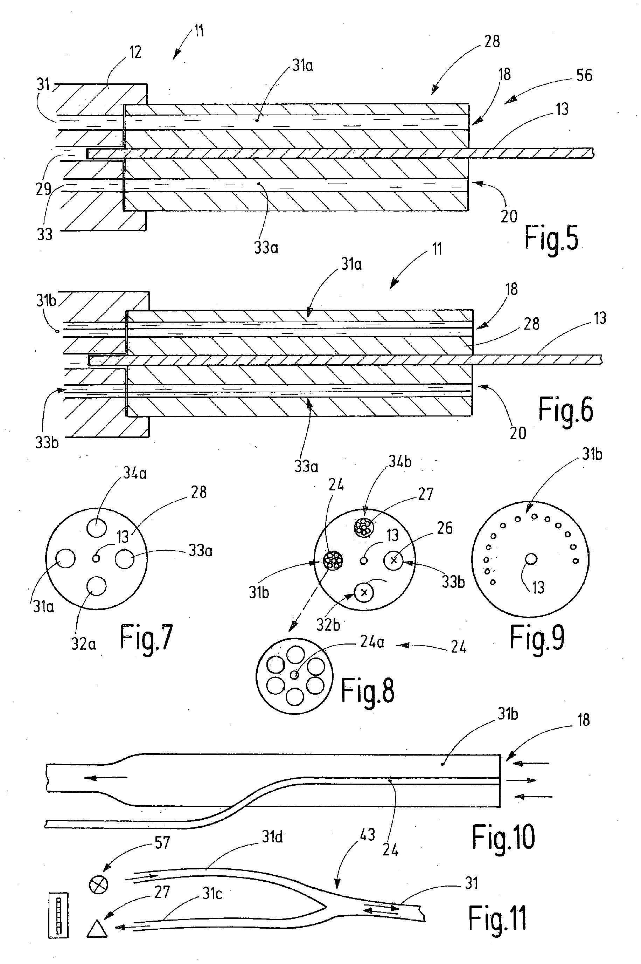

Die Indikatoreinrichtung 23 kann, wie Figur 1 und 2 veranschaulichen, mehrere Indikatoren 24 bis 27 (siehe auch Figur 8) umfassen, über die zum Beispiel ein Lichtsignal abgegeben wird, um dem Anwender eine Orientierung zu geben. Dies kann schon bei einem Instrument 11 mit lediglich einem einzigen Lichteintrittsfenster 18 und einer angeschlossenen Lichtanalyseeinrichtung 17 dadurch erfolgen, dass die Indikatoren 24 bis 27 (deren Anzahl spielt hier keine Rolle) zum Beispiel dann ein Signal abgeben, wenn ein von dem Gewebe, in dem ein Schnitt zu führen ist, abweichendes Gewebe angetroffen wird. Dies kann durch Aufleuchten, Farbumschlag oder dergleichen signalisiert werden. Bei Ausführungsformen, die zwei oder mehrere Lichteintrittsfenster 18 bis 21 aufweisen, kann zusätzlich eine Richtungsinformation erzeugt werden. Figur 3 veranschaulicht dazu die Gewinnung von Richtungsinformation durch Festlegung verschiedener Lichtaufnahmefelder 18a, 20a der Lichteintrittsfenster 18 und 20. In Figur 4 sind in Draufsicht die verschiedenen Lichteaufnahmefelder 18a, 19a, 20a, 21a der vier Lichteintrittsfenster 18, 19, 20, 21 veranschaulicht. Sie gruppieren sich um die Elektrode 13 und überschneiden einander möglichst wenig oder nicht. Jedes Lichtaufnahmefenster 18 bis 21 ist über entsprechende Lichtleitmittel mit der Lichtanalyseeinrichtung 17 verbunden, die parallel oder über einen Multiplexer zyklisch nacheinander die einzelnen von den Lichteintrittsfenstern 18 bis 21 aufgenommenen Lichtspektren analysiert und mit einem vorab aufgenommenen oder aus einer Datenbank bezogenen Gewebespektrum (oder mehreren gewonnenen oder vorab gespeicherten Gewebespektren) vergleicht.

In einem besonders komfortablem Fall werden auf diese Weise für die vier Lichtaufnahmefelder 18 bis 21a vier Gewebespektren aufgenommen und fortwährend mit einem oder mehreren Referenzspektren verglichen. Entsprechend kann derjenige der Indikatoren 24 bis 27 aufleuchten oder einen Farbumschlag zeigen, der mit einem Gewebe in Berührung kommt, das nicht dem für die Schnittführung vorgesehenen Gewebe entspricht. Damit wird dem Anwender auf besonders komfortable Weise Richtungsinformation und somit eine Orientierungshilfe bei der Anwendung gegeben.

In Figur 5 ist eine zweiteilige Ausbildung des Instruments 11 veranschaulicht, bei der an dem Handgriff 12 ein auswechselbares Kupplungsstück 28 angeordnet ist. Dieses trägt die Elektrode 13, die über eine entsprechende Stecckontakteinrichtung mit einer Leitung 29 in Verbindung steht. Das Kupplungsstück 28 kann ein oder mehrere Lichtleiter 31a, 33a aufweisen, die mit den Lichtleitfasern 31, 33 koppeln. Die distalen Enden der Lichtleiter 31a, 33a bilden die Lichteintrittsfenster 18, 20 und sind Teil der Lichtaufnahmeeinrichtung 56. Figur 7 veranschaulicht die Anordnung mit vier Lichtleitern 31a bis 34a.

Wie Figur 6 und 8 veranschaulichen, kann anstelle einer monofilen Lichtleitfaser 31 bis 34 auch jeweils ein Faserbündel 31b bis 34b vorgesehen sein. In dieses können Indikatorfasern 24a eingebettet sein, die die Indikatoren 24, 25, 26, 27 bilden. Die distalen Enden der Indikatorfasern der Faserbündel 31b bis 34b, die in Lichtaustrittsfenstern enden, sind Teil der Lichtaufnahmeeinrichtung 56. Die Indikatoren 24, 25, 26, 27 dienen dazu, die Gewebeindikation in das Sichtfeld des Anwenders zu projizieren und bilden somit Projektionsmittel. Die Detailansicht von Figur 8 veranschaulicht dies. Es wird deutlich, dass das Lichteintrittsfenster 18 hier von einem Kranz einzelner Fasern gebildet wird, die um eine als Indikator 24 dienende Faser herum angeordnet sind. Der Indikator 24 soll dazu dienen, zum Beispiel farbiges Licht auf das entsprechende Lichtaufnahmefeld 18a zu projizieren, um den Gewebetyp kenntlich zu machen. Über die Lichteintrittsfenster wird das aus diesem Lichtaufnahmefeld 18a stammende, von dem HF-Funken generierte Licht, aufgenommen und der Analyse zugeführt. Die in Figur 6 dargestellten Lichtleiter 31a, 33a des Kupplungsstücks 28 können entsprechend ausgebildet sein. Es ist jedoch auch hier möglich, ein Kupplungsstück 28 gemäß Figur 5 vorzusehen. Außerdem können die einzelnen Fasern eines Faserbündels 31b nach Vorbild der Figur 9 zum Beispiel teilkreisförmig angeordnet sein. Andere Anordnungen sind möglich, um symmetrische oder unsymmetrische Lichtaufnahmefel-der zu erhalten.

Es ist auch möglich, das zur Gewebeindikation dienende in das Lichtaufnahmefeld geleitete Licht und das aus dem HF-Funken stammende Licht in einer einzigen Lichtleitfaser 31 zu führen, wie es Figur 11 zeigt. An einer von dem in Figur 11 rechts zu denkenden Lichteintrittsfenster 18 entfernten Stelle kann ein Faserkoppler 43 vorgesehen sein, der durch eine Y-Verzweigung der Lichtleitfaser 31 gebildet wird. Während ein erster Teil 31c der Lichtleiterfaser 31 zu dem Lichtanalysator 17 führt, führt ein zweiter Teil 31d der Lichtleiterfaser 31 zu einer Quelle 57 die den Indikator 24 mit einem Signal vorzugsweise einem Lichtsignal versorgt. Der Lichtanalysator 17 ist in Figur 11 schematisch durch ein Prisma zur Lichtzerlegung und eine Zeilenkamera schematisch angedeutet.

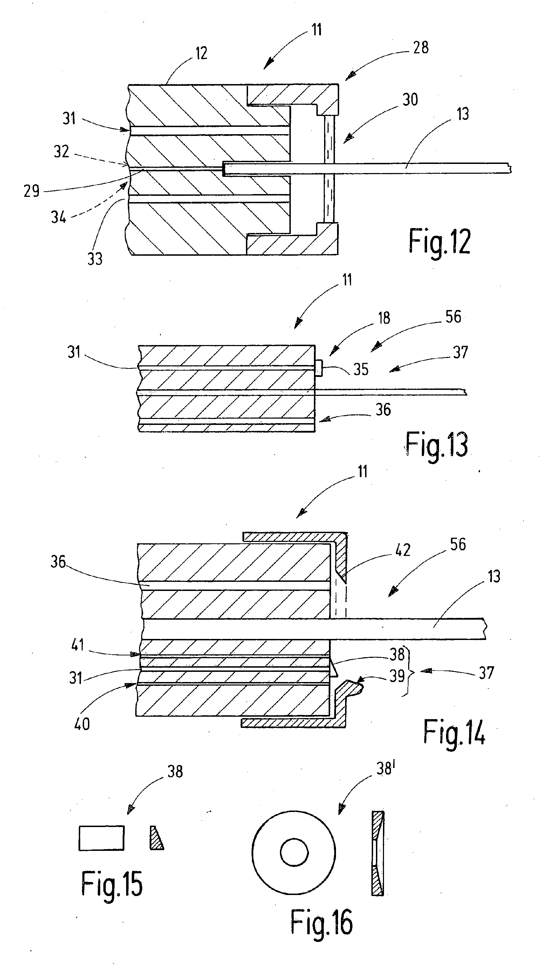

Der Aufbau des Instruments 11 kann auf vielfältige Weise variiert werden. Beispielsweise kann das Instrument 11, wie Figur 12 zeigt, an seinem distalen Ende ein Kupplungsstück 28 aufweisen, das die Elektrode 13 trägt und für den einmaligen Gebrauch vorgesehen ist. Es kann als Kappe auf das distale Ende des Instruments 11 aufgesteckt und mit diesem verbunden werden. Dabei gerät die Elektrode 13 in elektrischen Kontakt mit der Leitung 29 für elektrische Leistung, insbesondere HF-Spannung. Um die Elektrode 13 herum kann ein Sichtfenster 30 vorgesehen sein, um Licht des an der Elektrode 13 entstandenen Funkens zu einer oder mehreren Lichtleitfasern 31 bis 34 gelangen zu lassen. Zum Beispiel können die Lichtleitfasern 31 bis 34 an die Lichteintrittsfenster 18 bis 21 angeschlossen sein.

Das Kupplungsstück 28 mit der Elektrode 13 und dem Fenster 30 stellt ein Einmalprodukt dar. Der Handgriff 12 hingegen ist wieder verwendbar und entsprechend sterilisierbar.

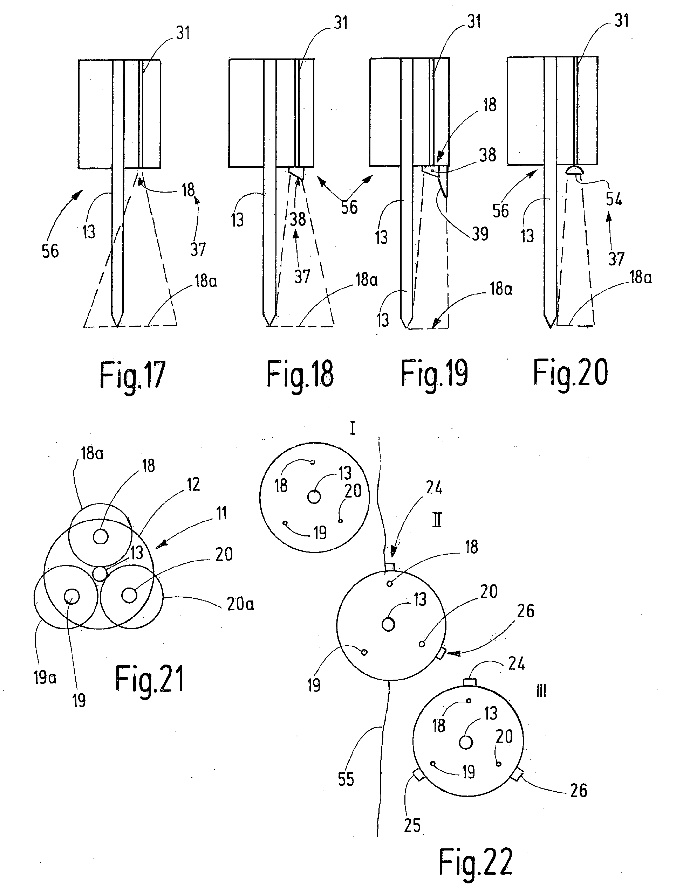

Figur 13 veranschaulicht eine abgewandelte Ausführungsform des Instruments 11 mit einer Lichtaufnahmeeinrichtung 56 mit einem oder mehreren Lichteintrittsfenstern 18. An dem distalen Ende steht mit der Lichtleitfaser 31 ein Silikonkissen 35 in Berührung, das zur Verringerung der Reflektionsverluste durch Anpassung der Brechungsindizes an der Kontaktstelle der optischen Fasern dient. Außerdem kann bei dieser, wie bei allen anderen, Ausführungsformen eine Reinigungseinrichtung beispielsweise in Form eines Fluidkanal 36 vorgesehen sein. Dieser dient der Zuführung von (flüssigem oder gasförmigem) Fluid zum distalen Ende des Instruments 11 und somit zur Verhinderung von Verschmutzung des Silikonkissens 35 oder jedes sonstigen Lichteintrittsfenster 18 bis 21 durch Rauchpartikel, Aerosol und Gewebestücke. Diese entstehen während der elektrochirurgischen Anwendung und könnten sich sonst auf der Stirnfläche der optischen Faser 31 oder sonstiger Elemente absetzen, die ein wie immer geartetes Objektiv 37 bilden. Als solches ist in der Ausführungsform nach Figur 13 lediglich das Silikonkissen 35 vorgesehen. Das Objektiv 37 kann jedoch, wie Figur 14 veranschaulicht, auch ein Prisma 38 enthalten, das an dem distalen Ende der Lichtleitfaser 31 angeordnet ist, um das Lichtaufnahmefeld auf einen bestimmten Bereich der Elektrode 13 zu richten. Zu dem Objektiv 37 können optional ein oder mehrere weitere Elemente gehören, wie beispielsweise ein seitlich in radialem Abstand zu der Elektrode 13 und dem Prisma 38 angeordneter Spiegel 39. Dieser kann, wie Figur 19 zeigt, dazu dienen, das Lichtaufnahmefeld 18a auf einen engen, der Elektrode 13 benachbarten Bereich, zu konzentrieren. Eine solche Objektivanordnung kann bei Ausführungsformen mit lediglich einem Lichteintrittsfenster 18, wie auch bei Ausführungsformen mit mehreren solchen Lichteintrittsfenstern, vorgesehen werden. Wie Figur 14 weiter veranschaulicht, können um die Lichteleitfaser 31 herum in ringförmiger Anordnung ein oder mehrere Fluidkanäle 40, 41 vorgesehen sein, um einen das Objektiv 37 sauber haltenden Fluidstrahl, beispielsweise einen Gasstrahl, zu erzeugen. Die Fluidkanäle 40, 41 können unabhängig von dem Fluidkanal 36 angeordnet sein. Dem Fluidkanal 36 kann eine Ablenkdüse 42 zugeordnet sein, um den aus dem Kanal 36 austretenden Strahl radial nach innen zu leiten. Das distale Ende des Fluidkanals 36, 40, 41, das Silikonkissen 35 und das Objektiv 37 sind auch Teil der Lichtaufnahmeeinrichtung 56.

Die Fluidkanäle 36, 40 und/oder 41 sind mit einer nicht weiter veranschaulichten Quelle (z.B. Pumpe oder Druckgasbehälter) für unter Druck stehendes Fluid verbunden. Zur Regulierung oder zumindest Sperrung und Freigabe des Fluidstroms sind ein oder mehrere Ventile vorgesehen. Diese sind vorzugsweise in dem Handgriff 12 angeordnet, um den Fluidstrom möglichst unverzögert ein und aus zu schalten. Das Ventil (bzw. die Ventile) können mechanisch oder elektrisch mit einem Aktivierungsschalter für die HF-Aktivierung verbunden sein, der z.B. ebenfalls an dem Handgriff angeordnet sein kann. Zwischen dem Aktivierungsschalter, dem HF-Generator und den Ventilen kann ein Verzögerungsblock vorgesehen sein, der sicher stellt dass die HF-Aktivierung erst dann erfolgt, wenn der Fluidstrom wie gewünscht aufgebaut ist.

Bei dieser Ausführungsform kann ein Keilprisma 38 nach Figur 15 zur Anwendung kommen oder ein Ringsprisma 38', das konzentrisch um die Elektrode 13 herum angeordnet und in Figur 16 gesondert im Querschnitt und in Draufsicht veranschaulicht ist. Die Spiegelfläche 39 dient der Umleitung des aufgenommenen Lichts zu dem Lichteintrittsfenster hin, das hier durch die Stirnfläche der Lichtleitfaser 31 gebildet ist. Dadurch wird eine hohe Lichtausbeute erhalten. Die Ablenkdüse 42 dient der Reinigung des Objektivs 37. Sie leitet den Fluidstrom radial nach innen um, so dass das Objektiv 37 gut vor Schmutz geschützt ist, wobei der Fluidstrom aber nicht direkt auf die Gewebeoberfläche geleitet wird. Dadurch können Irritationen des erzeugten Funkens sowie Gefahren, wie beispielsweise Gasembolien, vermieden werden. Der zusätzliche Fluidkanal 40, 41, der als koaxialer Spaltkanal ausgebildet sein kann, schützt das Keilprisma 38 sowie die Spiegelfläche 39 zusätzlich vor Verschmutzung.

Während die Ausführungsform des Instruments 11 nach Figur 14 schematisch in Figur 19 wiedergegeben ist, zeigen die Figuren 17, 18 und 20 andere Ausführungsformen, bei denen optional ebenfalls Kanäle 36, 40, 41 gemäß voriger Beschreibung vorgesehen sein können.

Bei der Ausführungsform nach Figur 17 wird das Sichtfenster 18 durch das Ende der Lichtleitfaser 31 gebildet. Das Lichtaufnahmefeld 18a erfasst den gesamten Umfang der Elektrode 13. Damit wird das von dem Funken der Elektrode 13 ausgehende Licht, unabhängig davon erfasst, in welcher radialer Richtung der Elektrode 13 das Gewebe liegt. Das Objektiv 37 wird hier nur durch die nahezu punktförmige Apertur der Lichtleitfaser 31 gebildet.

Bei der Ausführungsform nach Figur 18 gehört zu dem Objektiv 37 das Prisma 38, so dass ein Lichtaufnahmefeld 18a mit veränderter, beispielsweise reduzierter Größe entsteht. Das Lichtaufnahmefeld 18a liegt bezüglich der Elektrode 13 an lediglich einer Seite derselben. Dadurch wird eine merkliche Asymmetrie geschaffen, so dass vor allem das in Figur 18 rechts von der Elektrode 13 entstehende Licht aufgenommen wird. Diese Ausführungsform kann die Grundlage für Instrumente bilden, die mehrere Lichtaufnahmefelder 18a bis 21a festlegen, wie es in Figur 4 dargestellt ist.

Wie Figur 20 zeigt, kann das Objektiv 37 eine Kollimatorlinse 54 aufweisen, die vor dem distalen Ende der Lichtleitfaser 31 angeordnet ist. Die Kollimatorlinse oder ein anderes geeignetes Linsensystem kann in einem nicht weiter veranschaulichten Linsenhalter sitzen, der zum Beispiel auswechselbar ausgebildet sein kann. Außerdem kann die im Zusammenhang mit Figur 14 bereits erläuterte Ablenkdüse vorgesehen sein. Die Kollimatorlinse 54 kann zur Erzeugung eines nahezu parallelen Lichtstrahlenverlaufs genutzt werden, um gezielt ein kleines Lichtaufnahmefeld 18a und somit gezielt die Lichtaufnahme aus bestimmten Bereichen des HF-Funkens oder einer sonstigen elektrischen Entladung zu beziehen. Wiederum kann die Ablenkdüse dazu benutzt werden, die Lichtleitfaser 31 und/oder die Kollimatorlinse 54 vor Verschmutzung zu schützen, ohne den Fluidstrom auf die Gewebeoberfläche zu leiten.

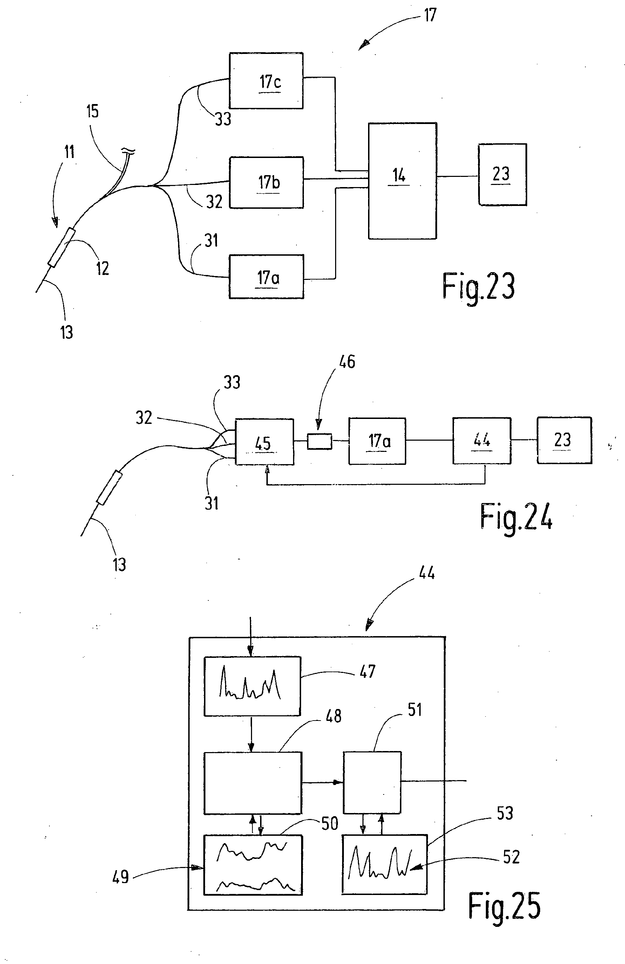

Figur 23 veranschaulicht die Systemarchitektur des Lichtanalysators 17. Der zu dem Lichtanalysator 17 führende Teil der Leitung 15 umfasst mehrere Lichtleitfasern 31, 32, 33, die jeweils zu unterschiedlichen Lichteintrittsfenstern gehören und somit Licht von unterschiedlichen Seiten der Elektrode 13 an die Lichtanalyseeinrichtung 17 heran führen. Diese umfasst ein, zwei oder mehrere Lichtanalysatoren 17a, 17b, 17c, die jeweils eine Spektralanalyse des aufgenommenen Lichts bewerkstelligen. Dazu können sie Gitterspektrometer oder Fotodiodenarrays aufweisen. Die Lichtanalyseeinrichtung 17 kann, wie in Figur 1 dargestellt, als externes Gerät oder auch integral im Handgriff 12 des Instruments 11 angeordnet sein. Ein Fotodiodenarray besteht aus mindestens zwei, vorzugsweise mehreren Fotodioden, die jeweils für einen geeigneten Wellenlängenbereich empfindlich sind. Damit kann entsprechend ein mehr oder weniger fein gerastertes Spektralsignal für das empfangende Licht erzeugt werden. Dieses wird einer nachgeschalteten Verarbeitungseinrichtung 44 zugeführt, die die Indikatoreinrichtung 23 ansteuert.

Anstelle dieser Anordnung mit drei Lichtanalysatoren kann die Anordnung gemäß Figur 24 auch einen einzigen Lichtanalysator 17a aufweisen, dem ein Multiplexer 45 und gegebenenfalls eine Intensitätserhöhungsvorrichtung 46 vorgeschaltet sind. Der Multiplexer 45 kann von der Verarbeitungseinrichtung 44 gesteuert sein, um nacheinander gezielt das Licht der einzelnen Lichtleitfasen 31, 32, 33 zu analysieren. Diese Anordnung ist besonders dann empfehlenswert, wenn eine große Zahl von Lichteintrittsfenstern vorgesehen ist, die auf verschiedene Bereiche eines Funkens blicken. Die Intensitätserhöhungsvorrichtung 46 ist beispielsweise eine Koppeleinrichtung, die auf eine Minimierung von Lichtverlust ausgerichtet ist, beispielsweise indem Licht optimal auf den Eingangsspalt eines Spektrometers verteilt wird. Dies kann durch eine geometrische Anpassung der herankommenden Lichtleitfaser vom Rundquerschnitt auf einen, Flachquerschnitt erfolgen. Wird anstelle einer monofilen Lichtleitfaser 31 ein Faserbündel verwendet, kann dies durch ein Aufspleißen des Faserbündels bewerkstelligt werden. Alternativ kann eine geeignete Fokussieroptik Anwendung finden, die beispielsweise Zylinderlinsen umfasst.

Figur 25 veranschaulicht die Betriebsweise der Verarbeitungseinrichtung 44 und somit deren Programmierung. An ihrem Eingang nimmt die Verarbeitungseinrichtung 44 das Spektrum 47 auf. Dieses liegt in Form geeigneter elektrischer Signale, d.h. Daten vor. Dieses Spektrum wurde an einem Lichteintrittsfenster 18 aufgenommen. Ein Komparatorblock 48 bildet eine Betriebssteuereinrichtung. Er vergleicht das Spektrum 47 mit vorgegebenen Spektraleigenschaften 49, die in einem Speicherblock 50 zum Abruf bereitgehalten werden. Bei den Spektraleigenschaften 49 kann es sich um die vorgegebenen Größen oder Größenbereiche von Spektrallinien oder Spektralbereichen handeln. Sie sind so festgelegt, dass sie nur dann auftreten, wenn der HF-Funke über der Elektrode 13 mit biologischem Gewebe in Berührung steht. Die Spektraleigenschaften sind so festgelegt, dass sie beispielsweise in HF-Funken, die in der Luft oder gegen Metallteile brennen, oder im Umgebungslicht nicht vorkommen. Beispielsweise kann dazu die gelbe Natriumlinie oder deren Verhältnis zur sonstigen Lichtintensität dienen.

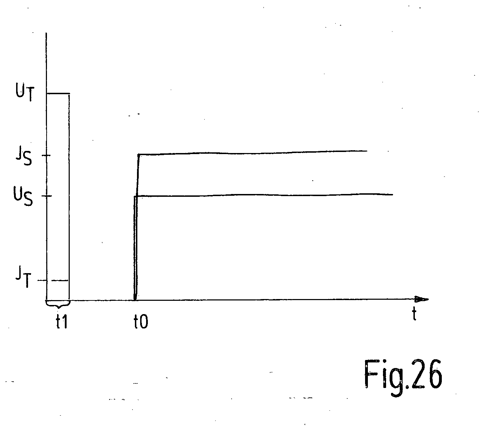

Stellt der Komparatorblock 48 fest, dass das aufgenommene Licht in bestimmten erforderlichen Eigenschaften mit den Spektraleigenschaften 49 übereinstimmt, gibt er das aufgenommene Spektrum 49 zur weiteren Analyse an den Analyseblock 51. Dieser vergleicht das Spektrum 47 nun mit einem oder mehreren Referenzspektren 52, die in Gestalt entsprechender Referenzdatensätze vorliegen. Diese können beispielsweise aus einer Datenbank stammen oder zu Anwendungsbeginn oder auch zuvor ermittelt worden sein. Figur 26 veranschaulicht die Aufnahme derselben. Beispielsweise wird in einem ersten Zeitbereich t1 ein Testfunken gesetzt. Dabei kann mit einer hohen U_{T} aber einem niedrigem Strom I_{T} gearbeitet werden, beispielsweise, um unerwünschte chirurgische Effekte zu vermeiden. Beispielsweise kann zu dem Zeitpunkt tl ein Referenzspektrum von gesundem Gewebe aufgenommen werden. Alternativ oder ergänzend kann zu einem weiteren Zeitpunkt ebenfalls mit erhöhter Spannung UT und vermindertem Strom 1T an einem anderen Gewebe ein Testfunken erzeugt und ein weiteres Referenzspektrum aufgenommen werden. Beispielsweise kann dies an malignem Gewebe geschehen.

Zu einem späteren Zeitpunkt t0 kann eine Anwendung mit niedrigerer Spannung U_{S} aber höherem Strom 1ₛ und somit dem gewünschten chirurgischen Effekt durchgeführt werden. Der Betrieb kann dabei wie in den Figuren 25 und 26 veranschaulicht erfolgen.

In Figur 21 ist ein Instrument 11 mit drei Lichteintrittsfenstern 18, 19, 20 veranschaulicht, die drei um die Elekrode 13 herum angeordnete Lichtaufnahmefelder 18a, 19a, 20a festlegen. Das dort aufgenommene Licht wird in der Lichtanalyseeinrichtung 17 nach Figur 23 bis 25 analysiert.

In einer Position I in Figur 21 befindet sich die Elektrode 13 in gesundem Gewebe. Die Lichtaufnahmefenster 18, 19, 20 erfassen Licht, das der an der Elektrode 13 entstandene HF-Funken in gesundem Gewebe erzeugt, dessen Referenzspektrum als Referenzdatensatz 53 vorhanden ist. Zunächst erkennt der Komparatorblock 48, dass in biologischem Gewebe gearbeitet wird und gibt somit die Spektralanalyse frei. Der Analyseblock 51 erkennt den Schnitt im gesunden Gewebe, so dass die Indikatoreinrichtung 23 nicht angesteuert wird.

In der nächsten Position II erreicht die Elektrode 13 eine Gewebegrenze 55 zwischen der bekannten Gewebeart und einer anderen Gewebeart, beispielsweise malignem Gewebe, für das kein Referenzdatensatz vorliegt. Während über das Lichteintrittsfenster 19 noch Licht aufgenommen wird, das von einem Funken beziehungsweise Funkenanteil stammt, der ausschließlich in gesundem biologischem Gewebe erzeugt wurde, nehmen die Lichteinrittsfenster 18 und 20 Licht auf, das von Funken beziehungsweise Funkenanteilen stammt, die in andersartigen, zum Beispiel malignem Gewebe, erzeugt worden sind. Entsprechend erkennt der Analyseblock 51 die Nichtübereinstimmung mit dem Referenzdatensatz und aktiviert die Indikatoren 24, 26. Wird der Schnitt weiter in Richtung malignen Gewebes (oder sonstigem fremden oder unbekannten Gewebes) geführt, ist die Elektrode 13 schließlich vollständig in diesem Gewebe angekommen. Über alle drei Lichteintrittsfenster 18, 19, 20 wird nun Licht aufgenommen, das nicht mit dem Referenzspektrum übereinstimmt. Entsprechend signalisieren alle Indikatoren 24, 25, 26 die Abweichung von dem im Kalibrierschritt typisierten Gewebe.

Es ist auch möglich, mit mehreren Referenzdatensätzen zu arbeiten, beispielsweise indem ein erster Referenzdatensatz für gesundes Gewebe und ein zweiter Referenzdatensatz für malignes Gewebe aufgenommen wird. In diesem Fall kann jeder der Indikatoren zum Beispiel durch Aufleuchten und/ oder Farbumschlag Information liefern, beispielsweise grün für gesundes Gewebe, rot für malignes Gewebe und eine weitere Farbe, zum Beispiel blau für unbekanntes Gewebe.

Diese Signale könne, wie in Figur 2 und 3 angedeutet, durch Indikatoren am Handgriff 12 oder in oder an dem Kupplungsstück 28 sowie auch durch Abstrahlung in die jeweiligen Lichtaufnahmefelder 18a bis 20a vorgenommen werden und liegen somit im Blickfeld des Anwenders.

Bei einer elektrochirurgischen Einrichtung 10 zur Anwendung an biologischem Gewebe wird durch spektrale Analyse des an der Elektrode 13 entstehenden Lichts zuvor typisiertes Gewebe erkannt. Eine akustische oder optische Indikatoreinrichtung 23 zeigt dauernd oder bei Erfassung bestimmter Gewebe den Gewebetyp an. Indikatoren, insbesondere optische Indikatoren 24 bis 27, sind im Anwendungssichtfeld angeordnet, um den Anwender bei der Schnittführung zu unterstützen.

### Bezugszeichenliste:

- 10: Einrichtung
- 11: Instrument
- 12: Handgriff
- 13: Elektrode
- 14: Gewebe
- 15: Kabel
- 16: Gerät
- 17: Lichtanalyseeinrichtung
- 17a-17c: Lichtanalysatoren
- 18 - 21: Lichteintrittsfenster, sie gehören zu einer Lichtaufnahmeeinrichtung 56
- 18a-21a: Lichtaufnahmefelder
- 22: Anwendungssichtfeld
- 23: Indikatoreinrichtung
- 24 - 27: Indikatoren, Projektionsmittel
- 28: Kupplungsstück
- 29: Leitung

- 30: Fenster
- 31 - 34: Lichtleitfasern
- 31a-34a: Lichtleiter
- 31b-34b: Faserbündel
- 31c: erster Teil der Lichtleitfaser 31 zum Lichtanalysator 17
- 31d: zweiter Teil der Lichtleitfaser 31 zur Quelle 57
- 35: Silikonkissen
- 36: Reinigungseinrichtung, Fluidkanal
- 37: Objektiv
- 38, 38': Prisma
- 39: Spiegel
- 40, 41: Gaskanal
- 42: Ablenkdüse
- 43: Faserkoppler

- 44: Verarbeitungseinrichtung
- 45: Multiplexer
- 46: Intensitätserhöhungsvorrichtung
- 47: Spektrum
- 48: Komparatorblock, Betriebsteuereinrichtung
- 49: Spektraleigenschaften
- 50: Speicherblock
- 51: Analyseblock
- 52: Referenzspektren
- 53: Referenzspektraldatensatz
- 54: Kollimatorlinse
- 55: Gewebegrenze
- 56: Lichtaufnahmeeinrichtung
- 57: Quelle, Lichtquelle
- 24a: Indikatorfaser

## Patentansprüche

1. Elektrochirurgische Einrichtung (10), insbesondere HF-Skalpell, zur Einwirkung auf ein biologisches Gewebe mittels elektrischer Entladung:
mit einem Instrument, das eine Elektrode (13) zur Erzeugung eines Funkens aufweist, die mit einer elektrischen Quelle (16) zur Speisung mit HF-Leistung verbindbar ist,
mit einer ersten Lichtaufnahmeeinrichtung (18), die ein erstes Lichtaufnahmefeld (18a) festlegt, das zumindest einen Teil des von der Elektrode (13) erzeugten Funkens erfasst,
mit einer Lichtanalyseeinrichtung (17) zur Ermittlung von Gewebemerkmalen durch Spektralanalyse des von den Lichtaufnahmeeinrichtungen (18) aufgenommenen Lichts,
**dadurch gekennzeichnet, dass** die Einrichtung eine Indikatoreinrichtung (23) zur Anzeige der Gewebemerkmale aufweist

2. Einrichtung nach Anspruch 1, **dadurch gekennzeichnet, dass** die Indikatoreinrichtung (23) einen akustischen Signalgeber umfasst.

3. Einrichtung nach Anspruch 1, **dadurch gekennzeichnet, dass** die Indikatoreinrichtung (23) im Anwendungssichtfeld (22) angeordnet ist.

4. Einrichtung nach einem der vorherigen Ansprüche, **dadurch gekennzeichnet, dass** sie eine zweite Lichtaufnahmeeinrichtung (19) aufweist, die ein zweites Lichtaufnahmefeld (19a) festlegt, das sich von dem ersten Lichtaufnahmefeld (18a) unterscheidet.

5. Einrichtung nach einem der vorigen Ansprüche, **dadurch gekennzeichnet, dass** die Lichtanalyseeinrichtung (17) dazu eingerichtet ist, das aufgenommene Licht mit mindestens einem Referenzspektraldatensatz (53) zu vergleichen.

6. Einrichtung nach Anspruch 5, **dadurch gekennzeichnet, dass** der Referenzspektraldatensatz (53) ein in einem oder mehreren Kalibrierschritten gewonnener Datensatz ist.

7. Einrichtung nach einem der vorigen Ansprüche, **dadurch gekennzeichnet, dass** die Indikatoreinrichtung (23) farbcodiert und/oder bezogen auf die Lage relativ zu der Elektrode (13) ortskodiert anzeigend ausgebildet ist.

8. Einrichtung nach einem der vorigen Ansprüche, **dadurch gekennzeichnet, dass** die Indikatoreinrichtung (23) Projektionsmittel (24, 25, 26, 27) aufweist, um der Gewebeindikation dienendes Licht in die Lichtaufnahmefelder (18a, 19a) zu projizieren.

9. Einrichtung nach Anspruch 8, **dadurch gekennzeichnet, dass** zu dem Projektionsmittel (24, 25, 26, 27) Indikatorfasern (24a) oder ein zweiter Teil (31d) einer Lichtleitfaser 31 gehören, die mit einem Lichtaustrittsfenster verbunden sind.

10. Einrichtung nach Anspruch 9, **dadurch gekennzeichnet, dass** die Lichtaustrittsfenster in oder an Lichteintrittsfenstern (18) der Lichtaufnahmeeinrichtung (56) angeordnet sind.

11. Einrichtung nach einem der vorigen Ansprüche, **dadurch gekennzeichnet, dass** die Lichtaufnahmeeinrichtung (56) die distalen Endbereiche wenigstens eines Faserbündels (31b) enthält, das sich von einem Lichteintrittsfenster (18) zu der Lichtanalyseeinrichtung (17) erstreckt.

12. Einrichtung nach einem der vorigen Ansprüche, **dadurch gekennzeichnet, dass** die Lichtaufnahmeeinrichtung (56) ein Lichteinrittsfenster (18) aufweist, dem eine Reinigungseinrichtung (36) zugeordnet ist.

13. Einrichtung nach einem der vorigen Ansprüche, **dadurch gekennzeichnet, dass** die Lichtaufnahmeeinrichtung (56) eine Objektivanordnung (37) enthält.

14. Einrichtung nach einem der vorigen Ansprüche, **dadurch gekennzeichnet, dass** die Elektrode (13) an einem Kupplungsstück (28) gehalten ist, das mit dem Instrument (11) lösbar verbunden ist wobei das Kupplungsstück (28) vorzugsweise ein oder mehrere Sichtfenster (30) aufweist, das vorzugsweise ringförmig um die Elektrode (13) herum angeordnet ist.

15. Einrichtung nach einem der vorigen Ansprüche, **dadurch gekennzeichnet, dass** die Lichtanalyseeinrichtung (17) eine Betriebssteuereinrichtung (48) aufweist, die dazu eingerichtet ist, die spektrale Gewebeanalyse zu starten, sobald die Lichtanalyseeinrichtung (17) eine festgelegte Spektraleigenschaft erfasst.

## Claims

1. Electrosurgical device (10), in particular an HF scalpel, for acting on a biological tissue by means of electrical discharge:
with an instrument having an electrode (13) for generating a spark, which electrode can be connected to an electrical source (16) for supplying HF power,
with a first light-receiving device (18) defining a first light-receiving field (18a) which receives at least part of the spark generated by the electrode (13),
with a light analysis device (17) for determining tissue features by spectral analysis of the light received by the light-receiving devices (18),
**characterised in that** the device comprises an indicator device (23) for displaying the tissue features.

2. Device according to claim 1, **characterised in that** the indicator device (23) comprises an acoustic signal emitter.

3. Device according to claim 1, **characterised in that** the indicator device (23) is arranged in the application viewing field (22).

4. Device according to any of the preceding claims, **characterised in that** it comprises a second light-receiving device (19) defining a second light-receiving field (19a) which differs from the first light-receiving field (18a).

5. Device according to any of the preceding claims, **characterised in that** the light analysis device (17) is configured to compare the received light with at least one reference spectral data set (53).

6. Device according to claim 5, **characterised in that** the reference spectral dataset (53) is a data set obtained in one or more calibration steps.

7. Device according to any of the preceding claims, **characterised in that** the indicator device (23) is configured for display with colour coding and/or location coding with reference to the position relative to the electrode.

8. Device according to any of the preceding claims, **characterised in that** the indicator device (23) has projection means (24, 25, 26, 27) for projecting light serving for tissue indication into the light-receiving fields (18a, 19a).

9. Device according to claim 8, **characterised in that** the projection means (24, 25, 26, 27) comprise indicator fibres (24a) or a second part (31d) of a light-conducting fibre (31) which are connected to a light outlet window.

10. Device according to claim 9, **characterised in that** the light outlet windows are arranged in or at light inlet windows (18) of the light-receiving device (56).

11. Device according to any of the preceding claims, **characterised in that** the light-receiving device (56) contains the distal end regions of at least one fibre bundle (31b) which extends from a light inlet window (18) to the light analysis device (17).

12. Device according to any of the preceding claims, **characterised in that** the light-receiving device (56) has a light inlet window (18) to which a cleaning device (36) is assigned.

13. Device according to any of the preceding claims, **characterised in that** the light-receiving device (56) contains a lens arrangement (37).

14. Device according to any of the preceding claims, **characterised in that** the electrode (13) is held on a coupling piece (28) which is releasably connected to the instrument (11), wherein the coupling piece (28) preferably comprises one or more viewing windows (30) arranged preferably in a ring around the electrode (13).

15. Device according to any of the preceding claims, **characterised in that** the light analysis device (17) comprises an operating control device (48) which is configured to start the spectral tissue analysis as soon as the light analysis device (17) detects an established spectral property.

## Revendications

1. Dispositif électrochirurgical (10), en particulier un scalpel HF, destiné à agir sur des tissus biologiques par le biais d'une décharge électrique :
comprenant un instrument qui présente une électrode (13), laquelle est destinée à générer une étincelle et peut être reliée à une source électrique (16) en vue d'une alimentation avec une puissance HF,
comprenant un premier dispositif de réception de lumière (18) qui définit une première zone de réception de lumière (18a), laquelle détecte au moins une partie de l'étincelle produite par l'électrode (13),
comprenant un dispositif d'analyse de lumière (17) destiné à déterminer des caractéristiques des tissus, par analyse spectrale de la lumière recueillie par les dispositifs de réception de lumière (18), **caractérisé en ce que** le dispositif présente un dispositif indicateur (23) destiné à afficher les caractéristiques des tissus.

2. Dispositif selon la revendication 1, **caractérisé en ce que** le dispositif indicateur (23) comprend un générateur de signaux acoustiques.

3. Dispositif selon la revendication 1, **caractérisé en ce que** le dispositif indicateur (23) est placé dans le champ visuel d'utilisation (22).

4. Dispositif selon l'une des revendications précédentes, **caractérisé en ce qu'**il présente un deuxième dispositif de réception de lumière (19) qui définit une deuxième zone de réception de lumière (19a), laquelle est différente de la première zone de réception de lumière (18a).

5. Dispositif selon l'une des revendications précédentes, **caractérisé en ce que** le dispositif d'analyse de lumière (17) est conçu pour comparer la lumière reçue avec au moins un ensemble de données spectrales de référence (53).

6. Dispositif selon la revendication 5, **caractérisé en ce que** l'ensemble de données spectrales de référence (53) est un ensemble de données obtenu en une ou plusieurs étapes de calibrage.

7. Dispositif selon l'une des revendications précédentes, **caractérisé en ce que** le dispositif indicateur (23) est réalisé de manière à effectuer un affichage avec un code couleur et/ou, en termes de position par rapport à l'électrode (13), avec un codage spatial.

8. Dispositif selon l'une des revendications précédentes, **caractérisé en ce que** le dispositif indicateur (23) présente des moyens de projection (24, 25, 26, 27) afin de projeter dans les zones de réception de lumière (18a, 19a), de la lumière servant à l'indication des tissus.

9. Dispositif selon la revendication 8, **caractérisé en ce que** le moyen de projection (24, 25, 26, 27) comprend des fibres d'indicateur (24a) ou une deuxième partie (31d) d'une fibre optique (31), qui sont reliées à une fenêtre de sortie de lumière.

10. Dispositif selon la revendication 9, **caractérisé en ce que** les fenêtres de sortie de lumière sont disposées dans ou sur des fenêtres d'entrée de lumière (18) du dispositif de réception de lumière (56).

11. Dispositif selon l'une des revendications précédentes, **caractérisé en ce que** le dispositif de réception de lumière (56) contient les parties d'extrémité distales d'au moins un faisceau de fibres (31b) qui s'étend à partir d'une fenêtre d'entrée de lumière (18) jusqu'au dispositif d'analyse de lumière (17).

12. Dispositif selon l'une des revendications précédentes, **caractérisé en ce que** le dispositif de réception de lumière (56) présente une fenêtre d'entrée de lumière (18) à laquelle est associé un dispositif de nettoyage (36).

13. Dispositif selon l'une des revendications précédentes, **caractérisé en ce que** le dispositif de réception de lumière (56) contient un agencement d'objectif (37).

14. Dispositif selon l'une des revendications précédentes, **caractérisé en ce que** l'électrode (13) est fixée à un élément d'accouplement (28) qui est relié de façon amovible à l'instrument (11), l'élément d'accouplement (28) présentant de préférence une ou plusieurs fenêtres (30), qui est de préférence disposée sous une forme annulaire autour de l'électrode (13).

15. Dispositif selon l'une des revendications précédentes, **caractérisé en ce que** le dispositif d'analyse de lumière (17) présente un dispositif de commande de fonctionnement (48) qui est conçu pour lancer l'analyse spectrale des tissus, dès lors que le dispositif d'analyse de lumière (17) détecte une propriété spectrale définie.
